# NEW EUROPEAN PATENT SPECIFICATION

(11) **EP 1 002 055 B2**
(45) Date of publication and mention of the opposition decision: **14.04.2010**
(45) Mention of the grant of the patent: 30.06.2004
(21) Application number: 98938765.9
(22) Date of filing: 07.08.1998
(51) Int. Cl.: C12N 7/02

(54) **RECOVERY OF VIRUS FROM CELL CULTURE USING A HYPERTONIC SALT SOLUTION**
RÜCKGEWINNUNG VON VIREN AUS ZELLKULTUREN DURCH VERWENDUNG EINER HYPERTONISCHEN SALZLÖSUNG
RECUPERATION DE VIRUS A PARTIR D'UNE CULTURE CELLULAIRE AU MOYEN D'UNE SOLUTION SALEE HYPERTONIQUE

(30) Priority: 07.08.1997 GB 9716611
(43) Date of publication of application: 24.05.2000
(73) Proprietor: Xenova Research Limited, Cambridge CB4 0WG (GB)
(72) Inventor: JOHNSTON, Michael, Denis, Cambridge CB4 4NY (GB); O'KEEFFE, Roderic, Simon, Essex SB11 3GA (GB)
(74) Representative: O'Brien, Caroline Jane
(86) International application number: PCT/GB1998/002387
(87) International publication number: WO 1999/007834

(56) References cited:
- EP-A- 0 573 107
- US-A- 5 204 257
- GÖTZE ET AL: "IMMUNOGENICITY AND PARTIAL PURIFICATION OF SOLUBLE H-2 ANTIGENS EXTRACTED WITH HYPERTONIC SALT" JOURNAL OF IMMUNOLOGY, vol. 112, no. 5, 1974, pages 1643-1651, XP002084064

## Description

### Field of the Invention:

This invention relates to the production of viruses and to the harvesting of virus preparations from virus-infected cell cultures, for example for experimental and therapeutic purposes, e.g. for the production of virus vaccines. In particular aspects the invention relates to methods and arrangements for the production of herpesviruses. Other aspects of the invention will be apparent from the description given below.

### Background of the invention and Prior Art

Several methods are known for producing live virus preparations, e.g. herpesvirus preparations, for vaccine and other purposes.

For example. US 3,985,615 (Osaka Res Foundation: T Kubo et al) shows production of live attenuated varicella virus for vaccine use by culture comprising passage in guinea pig primary embryonic tissue cells, US 5,024,836 (Merck; WJ McAleer et al) relates to production of lyophilized vaccine preparations based thereon.

DD-209738 (Cent Cerc Bioprep: IV Patrascu) illustrates production of another type of herpesvirus, for use as vaccine against Marek's disease is produced by (a) culturing specific-pathogen-free chicken embryo cells on dextran microspheres; (b) inoculating the culture at 80% confluence with turkey herpes virus strain FC-126 (clone 1. IIIb); (c) collecting the infected cells in SPGA medium (sucrose, phosphate, glutamate, bovine albumin fraction V) when the cytopathic effect is 80%; (d) subjecting the suspension to three ultrasonic pulses of 1 minute duration at 2 minute intervals and centrifuging it to recover a first crop of vaccine: (e) resuspending the sediment in SPGA medium and repeating step (d) to obtain a second crop of vaccine (to increase the vaccine yield by almost 20%); (f) freezing the combined vaccines at -100 deg.C prior to determining the virus titre; and (g) diluting with SPGA medium and freeze drying.

JP06234659-A (ZH Handai Biseibutsubyo Kenkyukai) describes, in an example, production of herpesviral vaccine on human diploid fibroblast MRC-5 cells cultured in MEM medium at 37 deg.C; comprising inoculation of varicella virus Oka strain seed virus at a MOI of 0.03 to MRC-5 cells and culture at 37 deg.C for 2 days. Virus is then suspended in a solution containing 6.4g NaCl, 0.16g KCl, 2.3g Na2HPO4..12H20, 0.16g KH2PO4, 50,0g sucrose, 1.0g Na L-glutamate, 2.0g gelatin, 25.0g gelatin hydrolysate and 0.1g EDA-3Na per 1.

EP 0 573 107. US 5,360,736 and US 5,607,852 (Merck: PA Friedman et al) describe processes for production of attenuated varicella zoster virus vaccine, including a process for preparing live, attenuated, cell-free varicella-zoster virus (VZV) vaccine that comprises: (a) Culturing VZV infection-susceptible cells, selected from human diploid cells, to confluency in monolayer culture, under conditions of sufficiently high nutrition to achieve a high degree of cell replication, and supplying a non-metabolizable disaccharide; (b) infecting the cells cultured according to step (a) at as close to the point of confluency as possible with as high a multiplicity of infection of VZV-infected cells as practical; (c) maintaining the VZV-infected culture in a state of high nutrition for about 22-96 hours and harvesting at the point of peak infectious VZV production; (d) washing the VZV-infected culture with a physiologic solution, optionally containing a lysosomotropic agent, such as ammonium chloride or chloroquine. prior to harvesting the VZV infected cells; (e) Harvesting the VZV infected cells into a minimal volume of a stabilizing solution and either disrupting the cells immediately or freezing the cells for later disruption; (f) Disrupting the VZV-infected cells to optimally release cell-associated VZV. and removing cellular debris, to provide a cell-free VZV preparation. The process discloses use of cell densities of up to ca. 500.000 cells/cm2 in conventional culture vessels. The process is proposed for mass production of live vaccine. Appropriate nutrient medium for growing cells in monolayer culture in that connection is described as consisting essentially of SFRE-2 medium supplemented with between 0.2 mg/mL and 0.4 mg/mL soybean lipid, the cells being selected from MRC-5 cells. WI-38 cells and Vero cells.

WO 92/05263 (Immunology Ltd; SC Inglis et al) and WO 94/21807 (Cantab Pharmaceuticals Research: Inglis et al) are illustrative of the provision of recombinant cells and cul ture methods for producing genetically disabled herpesvirus such as herpes simplex virus for vaccine purposes.

It remains desriable to provide methods for treatment of virus-containing preparations, capable of contributing to the manufacture of virus preparations in good yield and purity.

### SUMMARY AND DESCRIPTION OF THE INVENTION

According to one aspect of the present invention is provided a process according to claim 1. The salt solution can be contacted with the cell culture to yield a liquid containing useful virus content and a much reduced content of cells or cell debris by comparison with (for example) the product of ultrasonic disruption. This process can for example be particularly applicable to give an improved yield of virus for the manufacture of live virus vaccine in a case where otherwise a cell-disruption step might be used to harvest virus by disrupting virus-infected cells of a virus-producing cell culture.

Buffering and other constituents can be chosen suitably in accordance with normal practice for handling the viruses concerned.

The harvesting incubation can be carried out with gentle agitation. The cell culture to be treated to the harvesting incubation can be for example a monolayer culture or a microcarrier culture or a roller-bottle culture.

The harvesting salt solution can be buffered and maintained at a pH and temperature in themselves suitable for the culture of the virus-infected cells, e.g. about pH 7 and advantageously about 34 deg.C. for herpes simplex virus.

Contact time between the cultured cells and the harvesting liquid is not specially critical and can for example be in the range of about 2-24 hours. It has been found in connection with certain examples that for example about 4 hours contact time is preferable because it can offer good yield with acceptably low levels of cellular protein.

After contact between the cultured infected cells and the harvesting liquid, the liquid containing the harvested virus particles, can be separated by decantation or any other suitable method; the cultured cells themselves can be allowed to remain attached to the surface on which they were cultured, and can be discarded after the separation of the harvesting liquid.

The harvesting liquid can then if desired be treated by filtration and/or centrifugation to remove residual cells.

Desirably, the harvested preparation can be diluted or dia-filtered to approximately isotonic concentration. e.g. about 138 mM in sodium chloride.

According to a further aspect of the invention, the virus preparation harvested in this way can be treated with nuclease enzyme to reduce any content of contaminating nucleic acid to acceptable levels.

The diluted liquid can for example be treated with Benzonase (TM) nuclease enzyme, to degrade free nucleic acids (importantly DNA, and usually also RNA) at up to about 50 units/ml in the presence of about 2-10 mM magnesium ion, either for up to about 1 hour at from about 4 deg.C to room temperature.

The level of nuclease enzyme and other protein can then be reduced for example by dia-filtration against a suitable formulation buffer, through a membrane with a virus-retaining, e.g. 500kD, exclusion limit.

After these treatments the harvested virus can be transferred to a desired carrier liquid and frozen, lyophilised or otherwise stabilised in any suitable manner.

Processes according to examples of the invention can offer particular advantage in connection with highly celt-associated viruses, i.e. those viruses having a particularly high degree of cell association in culture, for example herpes simplex virus type 2 (HSV-2), pseudorabies virus (PRV), turkey herpesvirus and varicella zoster virus (VZV). With certain herpesviruses and culture conditions (e.g. with herpes simplex virus type 1 (HSV-1)) there can be a substantial spontaneous release of virus from the infected cells into the cell culture liquid, so that application of a process according to an example of the present invention can sometimes here be unnecessary and accordingly such examples of the invention are less preferred.

The invention can be applied with any appropriate adaptations of detail as will be readily accessible to those skilled in the art, to herpesviruses of various types, including for example wild-type herpes simplex virus and genetically disabled herpes viruses such as herpes simplex virus, and for example other herpes viruses as mentioned in the documents cited herein.

The virus preparations obtained by the use of processing steps as described herein can be further processed and made part of pharmaceutical compositions e.g. with per-se conventional ingredients of virus vaccines.

The invention is further described and illustrated by the following non-limitative example.

### EXAMPLE:

A process according to an example of the invention, for harvesting and purifying virus particles, can make use of a culture of Vero cells infected with HSV-2, grown essentially in known manner in conventional culture medium contained in roller bottles at about 100ml of medium per bottle. The culture medium, cell type and culture conditions can be for example as follows:

The Vero cells can be passaged at 2 x 10^7 cells per roller bottle. Culture can be carried out using DMEM medium with 4.5 g/l glucose without sodium pyruvate and with Glutamax-1 (TM) (L-alanyl-L-glutamine), 862 mg/l. Incubation can be carried out for example at about 37 deg.C and for about 120 hours (5 days). Confluent cell cultures can then be infected with HSV-2 at a multiplicity of infection of about 0.01, by diluting the virus in DMEM to the level where 1 ml is added to each roller bottle which is then returned to the roller-incubation apparatus at about 34-37 deg.C. When cytopathic effect is observed to be 80-100%, e.g. 65-72 hours after infection, the roller bottles can be treated as ready for virus harvest.

The culture medium can be decanted from each bottle and replaced by 10ml per bottle of a buffered sodium chloride solution (about 0.8-0.9M) containing 0.01M sodium citrate pH 7.0. The cells in the roller bottle in contact with this buffered sodium chloride solution can be rolled and incubated at about 34 deg.C for about 4 hours.

The cultured cells themselves in the roller bottle can largely remain attached to the bottle surface and can be discarded after separation of the liquid containing the harvested virus particles.

The liquid in the bottle, comprising the buffered saline and material from the cell culture in suspension, including virus, can be removed by pipette and centrifuged at about 3000 rpm in a Sorvall RT6000 (TM) centrifuge for about 10 minutes (e.g. at RCFmax about 1876). The cells in the pellet, and those remaining in the bottle, are discarded (under appropriate virus-containment conditions) and the supernatant is taken by pipette to the next step, which can be continuous flow centrifugation. Pre-filtration can be carried out with a 0.8 micron filter. The supernatant liquid from centrifugation can be diluted or diafiltered to a final concentration (in respect of sodium ion) of 138mM.

The diluted liquid can then be treated with Benzonase (TM) nuclease enzyme, to degrade free nucleic acids (importantly the enzyme used has DNase activity, and usually also. like Benzonase (TM), will have RNase activity) at up to about 50 units/ml in the presence of about 2-10 mM magnesium ion. e.g. for up to about 1 hour at a temperature from about 4 deg.C up to room temperature.

The reaction liquid can then be subjected to tangential cross-flow filtration (diafiltration) using a filter/membrane with a 500kD exclusion limit in a Filtron (TM) or other tangential crossflow device, using a recirculation rate of 1000 ml/min, a filtrate rate of 100 ml/min, and a backflush of 100 ml sodium citrate 0.01M pH 7.25 containing 138 mM sodium chloride.

The retentate from the cross-flow ultrafiltration step can then be treated by diafiltration against 5-10 volumes of citrate/saline buffer to reduce the amount of nuclease enzyme, and the retentate is finally subjected to 0.2 micron (sterilising) filtration optionally preceded by filtration with a filter of from about 0.45 micron to 5 Micron, using the same buffer again, after making the liquid containing the virus preparation up to 20 mg/ml in a suitable stabilising protein, preferably human serum albumin at about 20 mg/ml. It can be useful to prewash the filters with a liquid containing the same stabilising protein in the same buffer, before using the filters to treat the virus preparation.

The resulting product can be obtained as a suspension of virus particles in saline buffer and stabilising protein. in which the level of residual DNA can be satisfactorily low.

The yield from such a process has been found to be usefully good by comparison with a process involving ultrasonic cell disruption to liberate virus particles, followed by separation of virus particles from cell debris.

The invention can be very usefully applied, for example in a preferred embodiment carried out according to the example described above, to the culture and harvesting of genetically disabled HSV-2 virus for vaccine use, which virus has a deletion in respect of the gH gene essential for production of infectious new virus particles, and is culturable on a cell line which is based on Vero cells which have been made recombinant and able to express the viral gH gene which is missing from the viral genome, e.g. as described in specifications WO 92/05263 and WO 94/21807 (and see also A Forrester et al, J Viral 66 (1992) 341-348, also H E Farrell et al, J Virol 68 (1994) 927-932) and C McLean et al. J Infect Dis 170 (1994) 1100-1109).

It can also be preferable, according to convenience, to culture the cells and viruses on various forms of microcarriers in per-se known manner, instead of in roller bottles.

## Claims

1. A process of harvesting a herpesvirus from a cell culture infected therewith, which comprises treating said culture with a hypertonic aqueous salt solution in a harvesting incubation to yield a virus suspension, wherein the hypertonic aqueous salt solution is a solution of 0.8 to 0.9 M sodium chloride and wherein the process is without a cell-disruption step.

2. A process according to claim 1, wherein the virus suspension is further treated to formulate it as a pharmaceutical preparation suitable for use as a virus vaccine.

3. A process according to claim 1 or 2 wherein the harvesting salt solution is buffered at a pH about 7 and temperature about 34 deg.C. for the harvesting of herpes simplex virus.

4. A process according to any of claims 1-3 wherein the harvested preparation is then diluted or dia-filtered to approximately isotonic concentration.

5. A process according to any of claims 1-4 wherein the harvested virus preparation is treated with nuclease enzyme.

6. A process according to claim 5 wherein the preparation after nuclease treatment is dia-filtered against a formulation buffer, through a membrane with a virus-retaining exclusion limit.

7. A process according to any preceding claim wherein the harvested virus, after transfer to a desired carrier liquid, is frozen, lyophilised or otherwise stabilised.

8. A process according to claim 1, wherein the virus comprises herpes simplex virus type 2 (HSV-2), pseudorabies virus (PRV), turkey herpesvirus or varicella zoster virus (VZV).

## Patentansprüche

1. Verfahren zur Ernte eines Herpesvirus aus einer Zellkultur, die damit infiziert wurde, umfassend das Behandeln der Kultur mit einer hypertonischen wässrigen Salzlösung in einer Ernteinkubation, um eine Virussuspension zu erhalten, wobei die hypertonische wässrige Salzlösung eine Lösung von 0,8 bis 0,9 M Natriumchlorid ist und wobei das Verfahren ohne Zellaufschlussschritt erfolgt.

2. Verfahren nach Anspruch 1, worin die Virussuspension weiter behandelt wird, um sie als pharmazeutisches Präparat zu formulieren, das zur Verwendung als Virusvakzine geeignet ist.

3. Verfahren nach Anspruch 1 oder 2, worin die Erntesalzlösung auf einen pH von etwa 7 und eine Temperatur von etwa 34°C gepuffert ist, um das Herpes-simplex-Virus zu ernten.

4. Verfahren nach einem der Ansprüche 1 bis 3, worin das geerntete Präparat dann auf etwa isotonische Konzentration verdünnt oder diafiltriert wird.

5. Verfahren nach einem der Ansprüche 1 bis 4, worin das geerntete Viruspräparat mit einem Nucleaseenzym behandelt wird.

6. Verfahren nach Anspruch 5, worin das Präparat nach der Nucleasebehandlung durch eine Membran mit einer Virusrückhalteausschlussgrenze gegen einen Formulierungspuffer diafiltriert wird.

7. Verfahren nach einem der vorangegangenen Ansprüche, worin das geerntete Virus nach Übertragung in eine gewünschte Trägerflüssigkeit gefroren, gefriergetrocknet oder auf andere Weise stabilisiert wird.

8. Verfahren nach Anspruch 1, worin das Virus ein Herpes-simplex-Virus vom Typ 2 (HSV-2), ein Pseudo-Rabies-Virus (PRV), ein Truthahn-Herpesvirus oder ein Varicella-zoster-Virus (VZV) umfasst.

## Revendications

1. Procédé de récolte d'un virus de l'herpès d'une culture de cellules qui en est infectée, qui comprend le traitement de ladite culture avec une solution salée aqueuse hypertonique dans une incubation de récolte pour obtenir une suspension du virus, où la solution salée aqueuse hypertonique est une solution de 0,8 à 0,9 M de sodium chlorure, et où le procédé est sans étape de rupture cellulaire.

2. Procédé selon la revendication 1, dans lequel la suspension du virus est en outre traitée pour la formuler comme une préparation pharmaceutique apte à utiliser comme un vaccin du virus.

3. Procédé selon la revendication 1 ou 2, dans lequel la solution salée de récolte est tamponnée à un pH d'environ 7 et une température d'environ 34°C pour la récolte du virus de l'herpès simplex.

4. Procédé selon l'une quelconque des revendications 1-3, dans lequel la préparation récoltée est alors diluée ou dia-filtrée à une concentration approximativement isotonique.

5. Procédé selon l'une quelconque des revendications 1-4, dans lequel la préparation du virus récolté est traitée avec l'enzyme nucléase.

6. Procédé selon la revendication 5, dans lequel la préparation, après le traitement à la nucléase, est dia-filtrée contre un tampon de formulation, à travers une membrane avec une limite d'exclusion de rétention du virus.

7. Procédé selon l'une quelconque des revendications précédentes, dans lequel le virus récolté, après le transfert a un liquide porteur souhaité, est congelé, lyophylisé ou stabilisé d'une autre manière.

8. Procédé selon la revendication 1, où le virus comprend le virus de l'herpès simplex type II (HSV-2), le virus de la pseudo-rage (PRV), le virus de l'herpès du dindon ou le virus de la varicella zoster (VZV).
